Europäisches Patentamt

European Patent Office

Office européen des brevets

(19)

(11) Veröffentlichungsnummer : **0 184 121 B2**

# (12) NEUE EUROPÄISCHE PATENTSCHRIFT

(45) Veröffentlichungstag der neuen Patentschrift :
**30.03.94 Patentblatt 94/13**

(21) Anmeldenummer : **85115021.9**

(22) Anmeldetag : **27.11.85**

(51) Int. Cl.$^5$ : **A23L 1/30,** A23C 21/02,
A23L 2/38, A23L 1/236,
A61K 31/70, C13K 5/00,
C13K 13/00

(54) **Nahrungsmittel, Getränke, Genussmittel oder pharmazeutisches zum oralen Verabreichen bestimmtes Präparat, das Saccharose enthält.**

(30) Priorität : **04.12.84 CH 5767/84**

(43) Veröffentlichungstag der Anmeldung :
**11.06.86 Patentblatt 86/24**

(45) Bekanntmachung des Hinweises auf die
Patenterteilung :
**29.11.89 Patentblatt 89/48**

(45) Bekanntmachung des Hinweises auf die
Entscheidung über den Einspruch :
**30.03.94 Patentblatt 94/13**

(84) Benannte Vertragsstaaten :
**AT BE CH DE FR GB IT LI LU NL SE**

(56) Entgegenhaltungen :
**DE-A- 2 230 116**
**DE-A- 2 620 349**
**FR-A- 1 594 522**
**FR-A- 2 526 273**
**NL-A- 7 701 147**
**US-A- 3 852 496**
**Martindale: The Extra Pharmacopocia, (1982),
Seite 1712**
**Saure Milcherzeugnisse - Milchmischgetränke
und Desserts, H.J. KLUPSCH, Verlag Th.
Mann, Gelsenkirchen-Buer, (1984), S. 208-223**

(56) Entgegenhaltungen :
**Nizo-Mededelingen, no. 7, (1973), S. 90**
**Journal of Dairy Science, vol. 57, no. 8, 1974, S.
849-859**
**Byproducts from Milk, B.H. WEBB, The AVI
Publishing Company Inc., 1970, S. 66/67**
**Yoghurt, J.L. RASIC, Technical Dairy Publishing House, Copenhagen, 1978, S. 328**
**Milchwissenschaft 30 (12), 1975, S. 724-729**
**J. Dent. Res. 63, 1984, S. 1293-1297**
**Infection and Immunity, 36, 1982, S. 371-378**
**Infection and Immunity, 29, 1980, S. 981-989**
**Infection and Immunity, 31, 1981, S. 261-266**
**J. Peridontol. 54, 1983, S. 163-172**
**Archs. Oral. Biol., 14, 1969, S. 235-241**

(73) Patentinhaber : **Biodyn AG**
**Industriestrasse 31**
**CH-8305 Dietlikon (CH)**

(72) Erfinder : **Underberg, Emil**
**Industriestrasse 31**
**CH-8305 Dietlikon (CH)**
Erfinder : **Lembke, Andres**
**Eutiner Landstrasse 15**
**D-2420 Eutin-Sielbeck (DE)**

(74) Vertreter : **Goddar, Heinz J., Dr. et al**
**FORRESTER & BOEHMERT**
**Franz-Joseph-Strasse 38**
**D-80801 München (DE)**

EP 0 184 121 B2

## Beschreibung

Die Erfindung betrifft eine neuartige Verwendung von Galactose oder einer galactosehaltigen Zusammensetzung.

In der DE 2 620 349 A1 ist ein alkoholfreies Getränk beschrieben auf der Basis von Heidelbeer-Extrakt, das zur Süssung 50g Saccharose, 15 g Fructose und 35 g Galactose enthält.

Aus Klupsch, H.J. "Saure Milcherzeugnisse, Milchmischgetränke und Desserts", Verlag Th. Mann, Gelsenkirchen (1984), Seiten 208 bis 209 und Seiten 220 bis 223 sind Joghurtprodukte bekannt, die auf 100 Teile Saccharose Galactose in einem Mengenbereich von 1,17 bis 16,71 Teilen enthalten.

Aus US-PS 3 852 496 ist es bekannt, ein durch Hydrolysation von Lactose gewonnenes Gemisch aus Lactose, Glucose und Galactose als Süssungsmittel zum Ersatz von Saccharose einzusetzen.

Aus Martindale: The Extra Pharmacopoeia, (1982), Seite 1712, Position 12778 - b, ist es bekannt, Galactose für einen Leberfunktionstest einzusetzen.

Es ist bekannt, dass Zucker, vor allem Sacharose, die Bildung von Zahnkaries begünstigt. Da auf Saccharose in der menschlichen Ernährung nicht ohne Nachteile verzichtet werden kann, ist es Aufgabe der Erfindung, diese nachteilige Wirkung der aufgenommenen Saccharose zu verhindern. Diese Aufgabe wird durch die Verwendung von Galactose oder einer galactosehaltigen Zusammensetzung in einem Nahrungsmittel, Getränk, Genußmittel oder pharmazeutischen, zur oralen Verabreichung bestimmten Präparat, das Saccharose enthält, in einer Menge von 1,17 bis 16,71, vorzugsweise 10, Teilen Galactose auf 100 Teile Saccharose als zahnschonender Zusatzstoff zur Verminderung der kariogenen Wirkung der Saccharose gelöst.

Die Erfindung beruht auf der Entdeckung, dass die Galactose im Mundraum die Haftstellen oder Rezeptoren an Zähnen und Zahnfleisch besetzt und dadurch eine Besiedelung dieser Haftstellen mit den sacharoseabbauenden, karieserregenden Mikroorganismen vermindert oder verhindert. Ohne frei verfügbare Haftstellen können diese Mikroorganismen sich am Zahn nicht halten und mithin dort auch keine Karieswirkung ausüben.

Die Erfindung macht sich den Umstand zunutze, dass die angegebene Dosierung der Galactose ausreicht für die angestrebte Wirkung. Sie vermeidet stärkere Galactosedosierungen, die zu Verdauungsproblemen, Gaschmacksbeeinträchtigungen und erhöhten Kosten führen können.

Andere Zucker, wie Glucose und Fructose, begünstigen auch die Kariesbildung, wenn auch nicht in so hohem Masse wie Saccharose. Dem trägt eine Weiterbildung der Erfindung Rechnung, die Gegenstand des Anspruchs 2 ist.

Um zu zeigen, dass durch die Anwesenheit von Galactose die Besiedelung der Zahnoberfläche mit karieserregenden Mikroorganismen gehemmt wird, wurden Tierversuche mit Ratten durchgeführt. An die Ratten wurde in einer ersten Gruppe zu Vergleichszwecken während 42 Tagen eine 10%ige Saccharoselösung verabreicht. In der zweiten Gruppe hingegen wurde eine Lösung verabreicht, die 10% Saccharose und 1% Galactose enthielt.

Eine Überprüfung der Zahnoberflächen mit Indikatorfarbstoff und optischen Methoden zeigte, dass auf den Zähnen derjenigen Tiere, an welche die Lösung verfüttert wurde, die nur Saccharose enthlelt, grosse Mengen an zahnschädigenden Keimen, nämlich an Keimen von Streptococcus salivarius, Streptococcus mutans und Actinomyces species vorhanden waren. Im Gegensatz dazu waren die Zähne derjenigen Tiere, die eine Lösung erhielten, die neben der Saccharose noch 1% an Galactose enthielt, von diesen zahnschädigenden Keimen in wesentlich geringerem Ausmass besiedelt, und im allgemeinen war die Besiedelung auf 5% Besiedelungsdichte bis 20% der Besiedelungsdichte abgesunken, die bei der Vergleichsgruppe festgestellt wurde, an die die reine Saccharoselösung verabreicht worden war.

Es wurde versucht, ob ein zahnschonender Zusatzstoff, der zusätzlich zu der Galactose noch mineralische Bestandteile enthält, eventuell noch wirksamer bezüglich der Prävention von Zahnschäden ist als ein entsprechender zahnschonender Zusatz, der nur Galactose enthält.

Die durchgeführten Tests lassen darauf schliessen, dass erfindungsgemässe zahnschonende Zusatzstoffe, die zusätzlich zu Galactosen oder einer Mischung aus Galactose und Lactose noch mineralische Bestandteile enthalten, insbesondere Kalzium, Magnesium, Spurenmetalle und Phosphat, eine noch bessere, zahnschonende Wirkung zeigen, als entsprechende Zusatzstoffe, die keine mineralischen Bestandteile enthalten. Als Galactose wird in den erfindungsgemässen zahnschonenden Zusatzstoffen die in der Natur vorkommende D-Galactose eingesetzt, vorzugsweise $\alpha$-D-Galactose und die allenfalls zusätzlich anwesende Lactose ist auch die entsprechende in der Natur vorkommende Lactose, nämlich die 4-0-Beta-D-Galactopyranosyl-D-Glucose.

Man kann die Galactose durch Hydrolyse von Lactose, beispielsweise mit Hilfe verdünnter Schwefelsäure oder mit Hilfe von Enzymen oder durch die Einwirkung von Mirkoorganismen, gewinnen. Grosstechnisch einfach und kostengünstig durchführbar ist die Ausgestaltung nach Anspruch 4. Die Mikroorganismen beziehen dabei ihre Energie zum Teil durch Glucoseabbau, der durchaus wünschenswert ist, da dadurch die Galactose stärker angereichert wird. Die verbleibenden Anteile an Lactose und Glucose sind im Zusatzstoff nicht nachteilig, sie tragen zur Ernährung und zur Süssung bei und ihre nachteilige, kariogene Wirkung kann durch die Galactose kompensiert werden.

Auch die in der Molke enthaltenen Mineralsalze können beibehalten werden, weil sie nicht schädlich sind. Im Gegenteil, sie können der Demineralisierung der Zahnschmelzes entgegenwirken.

Der Einsatz des Lactobacillus leichmanii ist deshalb vorteilhaft, weil dieser Bazillus in der Lage ist, Lactose zu Galactose und Glucose zu spalten, ohne dabei die gebildete Galactose selbst zu metabolisieren.

Gemäss einer bevorzugten Ausführungsart verwendet man als Ausgangsstoff, der ausserdem noch die gewünschten Mineralstoffe enthält, Süssmolke, die pasteurisiert wurde, um die darin enthaltene Mikroorganismenflora abzutöten. Anschliessend wird dann diese pasteurisierte Süssmolke mit Lactobacillus leichmanii fermentativ teilweise abgebaut. Vorzugsweise wird dieser fermentative Abbau mit dem Lactobacillus leichmanii bei einer Temperatur im Bereich von 27-45°C durchgeführt. Man erhält so aus der Süssmolke ein entsprechendes Produkt, das Galactose, Flucose und Lactose und ferner noch die Mineralstoffe und Vitamine der Molke enthält.

Als Vitaminbestandteil enthält dieses Molkenprodukt beispielsweise Lactoflavin.

Das aus der pasteurisierten Süssmolke durch fermentativen Abbau mit Lactobacillus leichmanii gewonnene Produkt wurde zu einer angesäuerten Molkenpaste getrocknet und dieses Produkt wiederum auf die zahnschonende Wirkung getestet. Die Ergebnise waren sehr zufriedenstellend.

Bevorzugt sind saccharosehaltige Getränke, die einen pH-Wert von 5,4 aufweisen, den zahnschonenden Zusatzstoff enthalten und bei denen die im Getränk vorhandenen Säuren mindestens teilweise in Form der Natriumsalze oder Kalziumsalze vorliegen und die ferner als Puffermittel $Na_2HPO_4$ und/oder $CaHPO_4$ enthalten.

Nahrungsmittel oder Getränke enthalten den zahnschonenden Zusatzstoff vorzugsweise in einer Menge von 0,2 bis 3 Gewichtsprozent, vorzugsweise 0,5 bis 1,5 Gewichtsprozent, und speziell bevorzugt etwa 1 Gewichtsprozent, bezogen auf das fragliche Nahrungsmittel oder Getränk.

Es wurde ein Zusatzstoff getestet, der aus gleichen Teilen Galactose und Mineralstoffe aufgebaut war, wobei die mineralischen Bestandteile Kalium, Natrium, Kalzium, Magnesium und Phosphor waren und der Phosphorgehalt 0,5 bis 1%, berechnet als elementarer Phosphor, bezogen auf das Gesamtgewicht des Zusatzstoffes aus Galactose und Mineralstoffen, betrug.

Bei der Durchführung der Tests wurden drei Gruppen an Ratten untersucht:

An die Gruppe zu Vergleichszwecken wurde ein 10%ige wässrige Lösung von Saccharose verabreicht.

In der 10%igen Saccharoselösung wurde die saure Molkenpaste in einer solchen Menge gelöst, dass der Gehalt der Saccharoselösung an Galactose 1% betrug. Diese Lösung wurde an die erste Versuchsgruppe verabreicht.

In der 10%igen Saccharoselösung wurde ferner der Zusatzstoff aus Galactose und Mineralstoffen wieder in einer solchen Menge gelöst, dass die 10%ige Saccharoselösung 1% Galactose und 1% mineralische Bestandteile enthielt. Diese Lösung wurde an die zweite Versuchsgruppe verabreicht.

Die Versuchszeit betrug 42 Tage und am Ende dieser Versuchszeit waren bei der Gruppe zu Vergleichszwecken deutliche Zahnschädigungen feststellbar. Bei der ersten Versuchsgruppe waren kaum Schädigungen feststellbar und auch bei der zweiten Versuchsgruppe waren die Resultate ähnlich gut.

Es wurden einige im Handel erhältliche und in TABELLE 1 aufgeführte Getränke auf ihre kariogenen Eigenschaften geprüft. Das Getränk Nr. 1 zeigte dabei die geringsten Schäden und das Getränk Nr. 8 die grössten Schäden. Die übrigen Getränke zeigten Zwischenwerte für die Schäden, und zwar von Nr. 2 bis Nr. 7 in der Reihenfolge der Numerierung stärker werdend.

In der TABELLE 1 sind die Analysenergebnisse der getesteten Getränke angegeben und die dort angeführten Werte sind die Mittelwerte, die sich aus drei Einzelanalysen ergeben.

Tabelle 1

| Analyse | Getränk Nr. | | | | | | | |
|---|---|---|---|---|---|---|---|---|
| | 1 | 2 | 3 | 4 | 5 | 6 | 7 | 8 |
| Trockensubstanz % | 12,8 | 9,0 | 7,9 | 8,3 | 8,5 | 7,9 | - | 9,9 |
| Asche % | 0,13 | 0,26 | 0,03 | 0,03 | 0,04 | 0,05 | - | 0,05 |
| Saccharose % | 0,7 | 2,4 | 2,1 | 2,0 | 1,9 | 2,2 | 10,0 | 3,9 |
| Glucose % | 3,6 | 3,8 | 3,4 | 3,8 | 3,6 | 3,6 | ∅ | 3,5 |
| Fructose % | 9,1 | 2,4 | 1,9 | 2,0 | 2,5 | 2,0 | ∅ | 3,4 |
| Natrium mg/l | 72 | 607 | 48 | 44 | 38 | 38 | 24 | 76 |
| Kalium mg/l | 595 | 174 | 150 | 112 | 150 | 7 | 4,0 | 3 |
| Kalzium mg/l | 314 | 162 | 93 | 95 | 88 | 73 | 84 | 37 |
| Magnesium mg/l | 56 | 131 | 22 | 21 | 21 | 13 | 12,2 | 7 |
| Zink mg/l | 0,37 | 0,40 | 0,23 | 0,24 | 0,31 | 0,21 | 0,18 | ∅ |
| Kupfer mg/l | 0,01 | 0,08 | 0,02 | 0,04 | 0,04 | 0,03 | ∅ | 0,01 |
| Mangan mg/l | 0,49 | 0,33 | 0,01 | 0,02 | 0,07 | ∅ | ∅ | ∅ |

Tabelle 1 (Fortsetzung)

| Analyse | Getränk Nr. | | | | | | | |
|---|---|---|---|---|---|---|---|---|
| | 1 | 2 | 3 | 4 | 5 | 6 | 7 | 8 |
| Eisen mg/l | 31 | 0,35 | 0,20 | 0,16 | 0,48 | 0,14 | ∅ | 0,04 |
| Phosphat mg/i | 81 | 1885 | 13 | 25 | 25 | 646 | ∅ | 512 |

∅ = unterhalb der Nachweisgrenze
Getränk Nr. 1 = Fruchtsaft-Diätgetränk
Getränk Nr. 2 = Fruchtsaft-Schulgetränk
Getränk Nr. 3 = Orange-Fruchtsaft
Getränk Nr. 4 = Kirsch-Zitrone
Getränk Nr. 5 = Apfel-Zitrone
Getränk Nr. 6 = Cola-Zitrone
Getränk Nr. 7 = 10%ige Saccharoselösung
Getränk Nr. 8 = XXX-Cola

Wenn man ein Saccharose enthaltendes alkoholfreies Getränk unter Zugabe eines erfindungsgemässen zahnschonenden Zusatzes herstellt, dann ist es vorteilhaft, wenn der pH-Wert des Getränkes so eingestellt wird, dass er über einem pH-Wert von 5,4 liegt und dass ferner die Pufferkapazität des Getränkes zwischen den pH-Werten 6,0 und 7,0 erhöht wird, damit beim Konsum eines derartigen Getränkes ein Absinken des pH-Wertes in der Mundhöhle unter einen Wert von 6,5 möglichst vermieden wird. Ferner ist es vorteilhaft, den Mineralstoffgehalt des Getränkes, insbesondere den Gehalt an Kalziumionen, Kaliumionen und Magnesiumionen sowie an Phosphationen zu erhöhen.

Die Erfindung wird nun anhand von Beispielen näher erläutert:

Beispiel 1

Herstellung eines zahnschonenden Zusatzstoffes aus Molkenpulver

Das als Ausgansmaterial eingesetzte Molkenpulver hatte die folgende Zusammensetzung:

| Bestandteil | Menge in Gew.-% |
|---|---|
| Zuckerbestandteile | 72 |
| Proteine | 12 |
| Mineralstoffe | 8,5 |
| Milchsäure | 2,3 |
| Wasser | 5,2 |

Der Gehalt dieses Produktes an Mineralstoffen wurde getestet, und man erhielt dabei die folgenden Werte, jeweils ausgedrückt in %, bezogen auf 100 g Molkenpulver. Die Ergebnisse sind in der folgenden Tabelle zusammengestellt:

| Mineralischer Bestandteil | Gew.-%, bezogen auf das Molkenpulver |
|---|---|
| Kalium | 1,9 |
| Natrium | 1,3 |
| Calcium | 0,9 |
| Magnesium | 0,2 |
| Phosphor | 0,6 |

Als weitere mineralische Bestandteile waren Spurenmengen an Eisen, Kupfer, Zink und Nickel vorhanden.

Ausser dem Molkenprotein enthielt das Molkenpulver Aminosäuren und ferner die Vitamine A, E, C, $B_1$, $B_2$ und Pantothensäure.

Die Zuckerbestandteile des Molkenpulvers bestanden zu 90% aus Lactose, zu 4% aus Galactose und zu 6% aus Glucose.

Um den Gehalt des Produktes an Galactose zu erhöhen, wurde ein Teil Molkenpulver mit 3-4 Teilen Wasser vermischt. Man erhielt eine trübe Flüssigkeit. Diese wurde dann zur Entfernung von gegebenenfalls anwesenden Keimen sterilisiert.

Anschliessend beimpfte man die trübe Flüssigkeit mit einer Kultur von Lactobacillus leichmannii und bebrütete sie einen Tag lang bei 28°C.

Als Beispiel für Stämme von Lactobacillus leichmannii, die mit Vorteil zur Durchführung dieses Gärverfahrens herangezogen werden können, seien die Stämme Lactobacillus leichmannii mit den Hinterlegungsnummern DSM 20076 und DSM 20355 genannt, die bei der deutschen Sammlung von Mikroorganismen in D-3400 Göttingen, Griesebachstrasse 8, hinterlegt sind und die von dort auf Anfrage bezogen werden können.

Nach der Fermentation mit dem Lactobacillus leichmannii wurde das erhaltene Produkt auf die Zusammensetzung der Zuckerbestandteile getestet:

Man erhielt dabei die folgenden Ergebnisse, berechnet auf 100% Zuckerbestandteile:

| Zucker | Gew.-%, bezogen auf 100% der Zuckerbestandteile |
|---|---|
| Lactose | 40% |
| Galactose | 45% |
| Glucose | 15% |

Man sieht aus diesen Ergebnissen, dass der zum Lactoseabbau eingesetzte Mikroorganismus die bei der Hydrolyse gebildete Glucose weiter abbaute, während die gebildete Galactose nicht angegriffen wurde. Man erhielt so ein Produkt, das im Vergleich zu dem eingesetzten trockenen Molkenpulver eine sehr stark erhöhten Gehalt an Galactose und einen niedrigen Gehalt an Lactose besass.

Die nach der Fermentation mit dem Lactobacillus leichmannii erhaltene trübe Flüssigkeit wurde einer Gefriertrocknung unterworfen, wobei man ein festes lagerfähiges Pulver erhielt, das direkt als Zusatz zu saccharosehaltigen Nahrungsmitteln und Getränken zur Verhinderung von Zahnschäden verwendet werden kann.

Beispiel 2

Herstellung eines zahnschonenden Zusatzstoffes aus Süssmolke

Die frische Süssmolke wurde aus einer Käserei bezogen. Es war vorher aus Vollmilch durch Labzugabe ein Koagulat hergestellt worden und die Molke von dem festen Milchkoagulat abgepresst worden.

Die frische Süssmolke wurde pasteurisiert um die darin enthaltene Mikroorganismenflora abzutöten. Anschliessend wurde sie mit Lactobacillus leichmannii, beispielsweise einem der beiden im Beispiel 1 genannten hinterlegten Stämme bei einer Temperatur von 28°C während eines Tages bebrütet. Nach dieser Bebrütungszeit wurde die Flüssigkeit dann neu sterilisiert.

Diese sterilisierte Flüssigkeit kann entweder direkt verwendet werden, um ein entsprechendes alkoholfreies zahnschonendes Getränk herzustellen oder sie kann anschliessend getrocknet werden, wobei man ein Trockenpulver erhält, das direkt im Haushalt als zahnschonender Zusatzstoff zur Herstellung von saccharosehaltigen Getränken oder Saccharose und Stärke enthaltenden Nahrungsmitteln eingesetzt werden kann.

Beispiel 3

Herstellung eines Getränkepulvers

Ein nach dem Verfahren gemäss Beispiel 1 oder Beispiel 2 hergestelltes Trockenpräparat wurde mit der gleichen Gewichtsmenge an Saccharose vermischt. Zu dieser Mischung wurde, bezogen auf das Gesamtgewicht der Mischung, 2 Gew.-% an Zitronensäure und 2 Gew.-% an 2-basischem Calciumphosphat zugesetzt.

95 Gew.-% dieser Mischung wurden mit 5% Orangensaftpulver vermischt. Aromastoffe und Stabilisatoren wurden beigemengt.

Man erhielt so ein sofort lösliches Getränkepulver, das bei der Auflösung in Wasser ein Getränk liefert, welches aufgrund seines hohen Saccharosegehaltes nach dem Konsum den Blutzuckerspiegel steigert.

Durch Angabe spezieller Dosierungsvorschriften und eventuelle Zugabe von weiteren mineralischen Bestandteilen kann dieses Getränkepulver so angepasst werden, dass es zur Zubereitung eines isotonischen Getränkes geeignet ist.

Sogenannte isotonische Getränke haben sich in der letzten Zeit bei Sportlern immer mehr durchgesetzt, weil sie gut verträglich sind und durch ihren hohen Gehalt an mineralischen Bestandteilen und Zuckerbestandteilen, insbesondere Saccharose, rasch zu einer Kräftigung führen. In den entsprechenden erfindungsgemässen Produkten ist durch den Gehalt an Galactose die zahnschädigende Aktivität der Saccharose behoben worden.

Beispiel 4

Die nach dem Verfahren gemäss Beispiel 1 oder Beispiel 2 hergestellten Trockenprodukte wurden in der Menge von 1 Gew.-%, bezogen auf die Gesamtmenge der Mischung, zu stärkehaltigen oder saccharosehaltigen Produkten zugesetzt. Die verwendeten Produkte waren Puddingpulver, Pulver zur Herstellung von Dessertcremen und pulverförmigen Mischungen zur Herstellung von Kuchen. Es handelte sich dabei um im Handel erhältliche Produkte, die nach Zugabe von Milch und/oder Wasser und entsprechenden weiteren Behandlungsschritten, wie zum Beispiel kaltes Rühren und/oder Aufkochen und/oder Backen, dann die entsprechenden Fertigprodukte liefern.

Die erhaltenen Fertigprodukte waren bezüglich ihres Geschmackes und ihrer Konsistenz von entsprechenden Produkten, die ohne den erfindungsgemässen Zusatz zubereitet wurden, praktisch nicht unterscheidbar.

Die unter Verwendung der erfindungsgemässen Zusätze hergestellten fertigen Nahrungsmittel wurden ferner auf ihren Galactosegehalt hin geprüft. Es zeigte sich dabei, dass durch die angewandten weiteren Arbeitsschritte, wie Kochen und/oder Backen, praktisch kein Abbau der zugegebenen Calactose aufgetreten war.

Ferner wurden zu einem im Handel erhältlichen, entsprechende pharmazeutische Wirkstoffe enthaltenden Hustensirup ebenfalls die nach dem Verfahren gemäss Beispiel 1 oder Beispiel 2 hergestellten Trockenprodukte zugegeben, und zwar in einer Menge von 1 Gew.-% des Trockenproduktes, bezogen auf das Gewicht des Hustensirups. Auch in diesem Falle änderte sich der Geschmack und die Konsistenz des mit dem zahnschonenden Zusatzstoff versehenen Hustensirups im Vergleich zu dem entsprechenden Handelsprodukt praktisch nicht.

Als Beispiele für weitere saccharosehaltige pharmazeutische Präparate, bei denen durch die Zugabe der erfindungsgemässen Zusätze die zahnschädigende Aktivität gemildert werden kann, seien saccharosehaltige Umhüllungen von Tabletten oder Pastillen genannt und ferner Lutschtabletten, die zur Desinfektion des Mund- und Rachenraumes eingesetzt werden.

Beispiel 5

Die im Handel erhältlichen Getränke, deren Zusammensetzung weiter vorne in der Tabelle I angegeben ist, wurden verbessert, indem man ihnen 4,5 g des nach dem Verfahren gemäss Beispiel 1 bzw. des nach dem Verfahren gemäss Beispiel 2 hergestellten Molkenpulvers pro Liter zusetzte. Durch diese Zugabe erhöhte sich bei jedem der Getränke der Trockenrückstand um 4,2 g pro Liter.

Die Menge an Zuckerbestandteilen erhöhte sich durch die Zugabe bei jedem der Getränke um 2,6 g pro Liter.

Ferner erhöhte sich die Menge an Proteinen bei jedem der Getränke um 0,6 g pro Liter und die Menge der Mineralstoffe pro Liter um 0,4 g.

Die fraglichen Getränke wurden wieder 42 Tage lang an Ratten verfüttert und anschliessend wurden die Ratten auf Zahnschädigungen getestet. Es zeigte sich, dass die mit den Zusätzen gemäss Beispiel 1 und Beispiel 2 versehenen Produkte deutlich geringere Zahnschäden hervorriefen, als die entsprechenden Produkte ohne diese Zusätze.

Die mit den Zusätzen versehenen Fruchtsaftgetränke der Versuchsgruppe Nr. 1 und Fruchtsaftschulgetränke der Versuchsgruppe Nr. 2 riefen bei den Tieren praktisch keine Zahnbelagsbildung hervor.

Auch bei den Fruchtsaftgetränken der Versuchsgruppe Nr.3, 4, 5 und 6 zeigte sich durch Zugabe der Zusätzt gemäss Beispiel 1 und Beispiel 2 eine deutliche Verbesserung.


**Patentansprüche**

1. Verwendung von Galactose oder einer galactosehaltigen Zusammensetzung in einem Nahrungsmittel, Getränk, Genußmittel oder pharmazeutischen, zur oralen Verabreichung bestimmten Präparat, das Saccharose enthält, in einer Menge von 1,17 bis 16,71, vorzugsweise 10, Teilen Galactose auf 100 Teile Saccharose als zahnschonender Zusatzstoff zur Verminderung der kariogenen Wirkung der Saccharose.

2. Verwendung nach Anspruch 1, dadurch gekennzeichnet, daß 0,85 bis 1,55 Teile Galactose auf 100 Teile übrigen Zucker enthalten sind.

3. Verwendung nach Anspruch 1 oder 2, dadurch gekennzeichnet, daß mineralische Bestandteile, nämlich Kalzium, Magnesium, Phosphat und Spurenmetalle enthalten sind, das vorzugweise, Kalium, Natrium, Kalzium, Magnesium und Phosphat in der gleichen Menge wie die Galactose enthalten sind, und zwar vorzugsweise mit einem Phosphorgehalt von 0,25 bis 0,5%, bezogen auf den Galactoseanteil.

4. Verwendung nach einem der vorangehenden Ansprüche, dadurch gekennzeichnet, daß der Zusatzstoff gewonnen ist aus einem Lactose enthaltenden Produkt, vorzugsweise Molke, dessen Lactose durch mikrobiellen Abbau, vorzugsweise unter Einsatz von Lactobacillus leichmannii, vorzugsweise der Stämme DSM 20067 oder DSM 20355, zumindest teilweise in Galactose und Glucose gespalten ist und dessen dabei erhaltene Glucose zumindest teilweise durch den mikrobiellen Aufbau aufgezehrt wird.

5. Verwendung nach einem der vorangehenden Ansprüche, dadurch gekennzeichnet, daß die Galactose in der in der Natur vorkommenden Form, nämlich als α-D-Galactose, enthalten ist.

6. Verwendung nach einem der vorangehenden Ansprüche in einem Getränk, dadurch gekennzeichnet, daß die Pufferkapazität derart erhöht ist, daß beim Konsum ein Absinken des pH-Wertes in der Mundhöhle unter einen Wert von pH 6,5 vermieden wird.

7. Verwendung nach einem der vorangehenden Ansprüche in einem Getränk, dadurch gekennzeichnet, daß der pH-Wert auf einen Wert über 5,4 eingestellt ist, vorzugsweise durch Zugabe von Kalziumcarbonat, neutralem Kaliumtartrat, neutralem Kalziumtartrat, Kalziummaleat, Natriummaleat, Natriumhydrophosphat und/oder Kalziumhydrophosphat.

8. Verwendung nach einemder Ansprüche 1 bis 5 in einem pharmazeutischen Präparat, gekennzeichnet durch dessen Ausgestaltung als saccharosehaltige Umhüllung von Tabletten oder Pastillen oder zur Desinfektion des Mund- und Rachenraumes dienende Lutschtabletten.

EP 0 184 121 B2

## Claims

1.  Use of galactose or a composition containing galactose in a foodstuff, drink, delicacy or pharmaceutical product intended for oral administration and containing saccharose, in an amount of 1.17 to 16.71 and preferably 10 parts of galactose for 100 parts of saccharose, as a tooth-protecting additive for reducing the cariogenic effect of the saccharose.

2.  Use according to claim 1, characterized in that 0.85 to 1.55 parts of galactose are contained for 100 parts of other sugars.

3.  Use according to claim 1 or 2, characterized in that mineral constituents, namely calcium, magnesium, phosphate and trace metals are contained and that preferably potassium, sodium, calcium, magnesium and phosphate are contained in the same quantity as the galactose and preferably with a phosphorus content of 0.25 to 0.5%, based on the galactose proportion.

4.  Use according to any of the preceding claims, characterized in that the additive is obtained from a product containing lactose, preferably whey, whose lactose is at least partly split into galactose and glucose by microbial decomposition, preferably using Lactobacillus leichmannnii, preferably strain DSM 20067 or 20355 and whose glucose is at least partly consumed by the microbial decomposition.

5.  Use according to any of the preceding claims, characterized in that the galactose is in the naturally occurring form, namely as $\alpha$-D-galactose.

6.  Use according to any of the preceding claims in a drink, characterized in that the buffer capacity is increased in such a way that on consumption the pH-value in the oral cavity is prevented from dropping below 6.5.

7.  Use according to any of the preceding claims in a drink, characterized in that the pH-value is set to above 5.4, preferably by adding calcium carbonate, neutral potassium tartrate, neutral calcium tartrate, calcium maleate, sodium maleate, sodium-hydrophosphate and/or calcium hydrophosphate.

8.  Use according to any of the preceding claims in a pharmaceutical product, characterized by it being in the form of a saccharose-containing coating for tablets or pastilles or sucking tablets used for disinfecting the mouth and throat.

## Revendications

1.  Utilisation - dans un produit alimentaire, une boisson, un stimulant ou une préparation pharmaceutique destinée à l'administration par voie orale, ces produits contenant du saccharose -, de galactose ou d'une composition contenant du galactose en une quantité égale à 1,17 à 16,71 parties, et de préférence à 10 parties, de galactose pour 100 parties de saccharose, comme additif protégeant les dents en vue de diminuer l'effet cariogène du saccharose.

2.  Utilisation selon la revendication 1, caractérisée par le fait que de 0,85 à 1,55 partie de galactose est contenue pour 100 parties d'autres sucres.

3.  Utilisation selon la revendication 1 ou 2, caractérisée par le fait que sont contenus des composants minéraux, à savoir du calcium, du magnésium, du phosphate et des métaux à l'état de traces, et que, de préférence, du potassium, du sodium, du calcium, du magnésium et du phosphate sont contenus dans la même quantité que le galactose, et ce, de préférence, avec une teneur en phosphore de 0,25 à 0,5 % rapportée à la teneur en galactose.

4.  Utilisation selon l'une des revendications précédentes, caractérisée par le fait que l'additif est obtenu à partir d'un produit contenant du lactose, et de préférence de petit-lait, dont le lactose est dissocié en galactose et en glucose, du moins partiellement, par décomposition microbienne, de préférence en utilisant du lactobacillus leichmannii, de préférence des souches DSM 20067 ou DSM 20355, et dont le glucose qui y est contenu est absorbé, du moins partiellement, par la décomposition microbienne.

8

5. Utilisation selon l'une des revendications précédentes, caractérisée par le fait que le galactose est contenu sous une forme présente dans la nature, à savoir sous forme de d-galactose-$\alpha$.

6. Utilisation selon l'une des revendications précédentes dans une boisson, caractérisée par le fait que la capacité de tamponnage est augmentée d'une manière telle que, lors de la consommation, on évite que la valeur du pH dans la cavité buccale ne diminue au-dessous d'une valeur du pH égale à 6,5.

7. Utilisation selon l'une des revendications précédentes dans une boisson, caractérisée par le fait que la valeur du pH est réglée à une valeur inférieure à 5,4, de préférence par addition de carbonate de calcium, de tartrate de potassium neutre, de tartrate de calcium neutre, de maléate de calcium, de maléate de sodium, d'hydrophosphate de sodium et/ou d'hydrophosphate de calcium.

8. Utilisation selon l'une des revendications 1 à 5 dans une préparation pharmaceutique, caractérisée par le fait qu'elle est mise sous la forme d'enveloppe contenant du saccharose pour des comprimés ou des pastilles, ou de pastilles à sucer servant à désinfecter la cavité bucco-pharyngienne.